(19) Europäisches Patentamt
European Patent Office
Office européen des brevets

(11) Publication number: **0 047 116**
**B1**

(12) **EUROPEAN PATENT SPECIFICATION**

(45) Date of publication of patent specification: **10.07.85**

(51) Int. Cl.$^4$: **A 61 K 7/06**

(21) Application number: **81303845.2**

(22) Date of filing: **24.08.81**

(54) **Article and method for conditioning hair.**

(30) Priority: **26.08.80 GB 8027572**

(43) Date of publication of application:
**10.03.82 Bulletin 82/10**

(45) Publication of the grant of the patent:
**10.07.85 Bulletin 85/28**

(84) Designated Contracting States:
**AT BE CH DE FR GB IT LI NL SE**

(56) References cited:
**US-A-3 442 692**
**US-A-3 954 113**
**US-A-3 992 336**
**US-A-4 149 551**

(73) Proprietor: **UNILEVER PLC**
**Unilever House Blackfriars P O Box 68**
**London EC4P 4BQ (GB)**
(84) **GB**

(73) Proprietor: **UNILEVER NV**
**Burgemeester s'Jacobplein 1 P.O. Box 760**
**NL-3000 DK Rotterdam (NL)**
(84) **BE CH DE FR IT LI NL SE AT**

(72) Inventor: **Curry, Kenneth Vasey**
**36 Upton Park**
**Upton by Chester.**
**Chester (GB)**
Inventor: **Pike, Barry Graham**
**April Cottage Pipers Lane Lower Heswall**
**Wirral Merseyside (GB)**
Inventor: **Steer, Jean Ann**
**44 Lindsay Road New Haw**
**Weybridge Surrey (GB)**

(74) Representative: **Fransella, Mary Evelyn et al**
**Unilever PLC Patents Division P.O. Box 68**
**Unilever House**
**London EC4P 4BQ (GB)**

**EP 0 047 116 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to an article and a method for conditioning the hair, and a process for the manufacture of the article.

The frequent shampooing which is necessary to rid the hair of dirt and sebum tends to leave it tangled, harsh, lack lustre, full of static electricity and generally unmanageable, and it is well known to apply creme rinses or conditioners to the hair after shampooing to improve manageability and, in particular, wet combability. Such materials generally leave a film of resinous or waxy material on each hair shaft, thus increasing both lustre and manageability. Conditioners and creme rinses may contain *inter alia* cationic material which is substantive to the hair and which reduces the static charge on the hair; examples of such materials are given in US Patent Specification No. 3 472 840 (Union Carbide).

Conditioners and creme rinses are generally in liquid form and are applied to the hair subsequent to shampooing, rinsing, and towelling to a partially dry state. The conditioner must then be rinsed off and the hair generally allowed to dry without further towelling. This operation is cumbersome and time-consuming in itself and also increases the drying time of the hair, so that there is a tendency for the user, if pressed for time, sometimes to omit the conditioning stage altogether.

It is also difficult using a liquid conditioner to treat only a part of the hair, as may be desired for example in the case of long hair which is greasy near the roots but brittle and fly-away at the ends.

In US Patent Specification No. 4 149 551 and British Published Applications Nos. 2 022 415 and 2 024 623 there has been proposed a towel-type hair-conditioning article in the form of a flexible substrate, such as non-woven rayon, carrying a releasable coating of a solid, water-insoluble conditioning agent such as ditallow dimethyl ammonium chloride. When the article is rubbed on damp or wet hair, the coating of solid conditioning agent is transferred to the hair. This can either be rinsed off or left on as desired. As with conventional liquid conditioners, conditioning is preferably carried out after shampooing, rinsing and partial drying. With this type of article, difficulties have been experienced in achieving efficient transfer of the conditioner from the towel to the hair, and efficient distribution of the solid conditioning material over the hair. Furthermore, user surveys have shown the existence of a credibility gap in that some consumers are not altogether convinced that any active ingredient is actually present on the towel; the loading of conditioner on the towel has to be relatively low in order to prevent overloading of the hair, and these consumers feel that there is insufficient visual and/or tactile evidence of the presence of conditioning material on the towels.

The present invention provide an article for conditioning hair, which comprises a flexible water-insoluble and non-water-dispersible substrate impregnated with an aqueous solution or dispersion comprising one or more hair-conditioning agents and colouring matter and/or perfume, the weight ratio of water associated with the substrate to substrate being at least 1.5 to 1 and the amount of hair-conditioning agent present being from 2 to 15 grams per square metre of substrate, the impregnated sheet being sealed within a water-impermeable container.

The invention also provides a method for the manufacture of an article according to the previous paragraph, which comprises so impregnating a flexible water-insoluble and non-water-dispersible substrate with an aqueous solution or dispersion comprising one or more hair-conditioning agents and colouring matter and/or perfume, that in the impregnated article thus obtained the weight ratio of water associated with the substrate to the substrate is at least 1.5 to 1 and the total amount of hair-conditioning agent present is from 2 to 15 grams per square metre of substrate, and subsequently sealing the impregnated sheet within a water-impermeable container.

A further aspect of the invention is a method for conditioning hair, which comprises rubbing the hair with an article as previously defined, whereby an effective amount of hair-conditioning agent is transferred from the article to the hair.

The hair may be wet, damp or dry. Optionally the conditioner may subsequently be rinsed off but this is by no means essential.

It will be noted that the article of the invention is wet, that is to say, it has associated with it a substantial amount of water in which the active hair-conditioning ingredient(s) is or are dissolved or dispersed, according to solubility. If necessary, depending on the active ingredients chosen, a minor amount of a cosmetically acceptable alcohol, preferably ethanol, may be included to assist in the dissolution or dispersion of the active ingredients.

The wet article of the present invention possesses various advantages over an article having a solid coating. Efficient transfer of the conditioning agent from the article to the hair is assured, and the dissolved or dispersed form of the active ingredient allows it to be distributed as evenly as possible over the hair.

Furthermore, the article is as suitable for application to dry or nearly dry hair as it is for the treatment of damp or wet hair. If an article carrying a solid coating of conditioner is applied to dry hair, uneven distribution of the conditioner will result, with overloading in some areas, leading to lankness and greasiness, and no conditioning at all in other areas. The article of the present invention, however, has sufficient wetness built in for effective treatment of dry hair without separate wetting being required. The article may accordingly be applied not merely immediately after shampooing but whenever desired, for example when atmospheric conditions cause a build-up of static charge on the hair, and it may also be used to treat just a part of the hair, as in the previously mentioned case of long hair greasy near the roots

and brittle and fly-away at the ends. The article of the invention can in this case be applied just to the ends of the hair without wetting and without any need to rinse the conditioner off; this is a very quick and simple process.

Consumer tests have also shown a user preference for the wet article of the invention; consumers are apparently more prepared to believe that an active ingredient is present when the article is wet. The smooth feel of the liquid on the article is readily associated in the consumer's mind with the feel of conditioner on the hair, and after use the exhausted article becomes quite different in appearance and feel. This "cueing" is assisted by the inclusion of small amounts of colouring matter and perfume in the impregnating liquid so that during use the article will lose colouring and perfume.

The article of the invention is based on a flexible substrate. This must of course be sufficiently absorbent to retain an adequate quantity of impregnating liquid, and is preferably of open or porous structure. The substrate must also have sufficient wet strength to retain its integrity during both storage and use. Suitable materials include woven and non-woven fabrics, high-wet-strength absorbent papers and other fibrous materials both natural and synthetic, sponges, and foamed plastic materials. The substrate may for example take the form of a sheet, but other shapes, for example, pads, or mittens to fit over the hand, are also possible provided that they allow convenient application of the conditioner to the hair.

Articles based on a sheet substrate, hereinafter referred to as towels, have proved most convenient, and preferred materials for the sheet substrate are non-woven fabrics, for example, "Vilene" (Trade Mark), a non-woven rayon. The sheet is preferably approximately hand-sized for convenience of manipulation; a square sheet 13 cms×13 cms has been found in consumer tests to be comfortable to use. A finger slit is advantageously provided to prevent the towel from slipping out of the user's hand. The loading of active ingredient on the towel is from 2 to 15 grams per square metre of substrate, calculated as concentrate. Below this level the amount of conditioner is simply too low to have a perceptible effect on the hair; above the upper limit the hair will be grossly overloaded with conditioner and will be left limp and greasy, especially if the hair is not subsequently rinsed. The preferred level of active ingredient is from 2 to 12 grams per square metre, more preferably 4 to 10 grams per square metre and desirably 6 to 9 grams per square metre.

The non-woven rayon fabric "Vilene" referred to above has a weight per unit area of approximately 92.9 grams per square metre. With this substrate, the loading range of from 2 to 15 grams per square metre is thus equivalent to a loading of from 2.15 to 16.15% by weight.

A wide variety of active ingredients may be used in the article of the invention, both singly and in admixture. Desirably at least one of the conditioning agents used is water-soluble, or at least soluble in water containing a minor proportion of alcohol, in order to take maximum advantage of the presence of the associated water; and desirably at least one of the conditioning agents used is a cationic material, because such materials show outstanding benefits in conditioning hair. Of course these two characteristics may be either combined in a single ingredient, or achieved by means of a suitable mixture of ingredients.

Advantageously the article of the invention includes a quaternary ammonium compound, a water-soluble one being especially preferred. Compounds of the formula.

$$R_1R_2R_3R_4N^+ X^-$$

are preferred in which $R_1$ is a $C_{10}$ to $C_{22}$ alkyl, $R_2$, $R_3$ and $R_4$ are lower alkyl and may be the same or different, and $X^-$ is a monovalent anion, especially a halide ion, or 1/m of an m-valent anion. An example of such a material with excellent hair-conditioning properties is cetyl trimethyl ammonium chloride, which is available commercially as a 50% aqueous solution under the trade name "Arquad 16—50".

Other quaternary ammonium compounds containing one or more quaternary nitrogen atoms and quaternary imidazolinium compounds may also be used in the article of the invention.

Advantageously the article may include a non-ionic material. Fatty alcohols, especially lauryl, cetyl and stearyl alcohols, have beneficial effects on hair, especially when used in conjunction with a cationic material. A mixture of cetyl and stearyl alcohols, sold commercially under the trade name of "Laurex CS", has proved valuable in this respect. Alternative non-ionic materials include silicones, sorbitan esters, polyhydric alcohol esters, tertiary phosphine oxides and tertiary amine oxides.

Accordingly, in a first preferred embodiment of the invention, a water-soluble cationic conditioning agent, preferably a quaternary ammonium compound, is used in conjunction with a fatty alcohol. The two components are advantageously used in approximately equal quantities. The fatty alcohol is of course substantially water-insoluble and is dispersed rather than dissolved.

Anionic materials, for example, alkyl sulphates, alkyl ether sulphates and alcohol sulphonates, may also be included in the article of the invention, either alone or in conjunction with other types of conditioner. Like the fatty alcohols, they are especially valuable when used together with a cationic material. In a second preferred embodiment of the invention, a water-soluble cationic conditioning agent, preferably a quaternary ammonium compound is used in conjunction with an alkyl sulphate (preferably $C_{10}$ to $C_{22}$), the cationic component being used in excess. The weight ratio of cationic conditioner to anionic conditioner may, for example, be in the range of from 2:1 to 5:1. In this case both components are water-soluble and will be in solution.

Zwitterionic and ampholytic hair-conditioning agents may also be used in the article of the invention.

As indicated previously, the article of the invention may be manufactured by impregnating a suitable substrate with a suitable aqueous conditioner composition containing appropriate concentrations of the desired active ingredients and suitable quantities of other materials such as colouring and perfume. Where the article is a towel, the sheet substrate may conveniently be dipped into a bath of suitable aqueous liquid, and excess liquid then removed, for example, by means of squeeze rollers.

To prevent the articles from drying out during storage, they need to be packed wet into water-impermeable containers. Sachets of plastic film and/or metal foil are ideal for this purpose. Preferably each article is sealed into an individual package. If necessary, towel-type articles can be folded or rolled for packing.

The following Examples illustrate the invention.

Example 1

A piece of "Vilene" non-woven rayon fabric (92.9 grams per square metre), approximately 13 cms×13 cms and weighing 1.57 grams, was provided with a slit about 3 cms long positioned centrally parallel to, and about 2 cms from, one edge, to serve as a finger slit. The piece of fabric was dipped in a bath containing an aqueous composition as follows:

| | Percentage by weight |
|---|---|
| 50% aqueous solution of cetyl trimethyl ammonium chloride ("Arquad 16—50") | 5.50 |
| Cetyl alcohol/stearyl alcohol mixture ("Laurex CS") | 2.75 |
| Perfume | 0.60 |
| Formalin (38% active) | 0.26 |
| Hexacol orange dye (1% solution) | 0.75 |
| Water to | 100.0 |

The pH of the composition had been adjusted to 3.0.

The wet towel was rolled gently to remove excess moisture, and packed wet in a polypropylene/aluminium foil sachet.

A number of towels were prepared in this way and the loading of the active conditioning ingredients (cetyl trimethyl ammonium chloride, cetyl alcohol and stearyl alcohol) on the towels ranged from 0.12 to 0.15 g per towel, equivalent to 7 to 9 $g/m^2$ or 7.6 to 9.6% by weight of the substrate. It will be noted that the weight ratio of cationic conditioning agent to fatty alcohol was 1:1.

In use, the towel was placed on the palm of the user's hand and the middle finger inserted through the slot. The towel could then be comfortably and conveniently be rubbed on the user's hair. It was found to impart conditioning benefits to both wet and dry hair. Best results were obtained on hair that had recently been shampooed; with wet, just shampooed hair wet combability was greatly improved.

Example 2

A number of towels were prepared by the procedure of Example 1, but using a bath containing a composition made up of the following ingredients:

| | Percentage by weight |
|---|---|
| "Arquad 16—50" | 5.50 |
| Lauryl alcohol | 2.75 |
| Perfume | 0.60 |
| Formalin (38% active) | 0.26 |
| Hexacol orange dye (1% solution) | 0.75 |
| Water to | 100.0 |

the pH of the composition again being adjusted to 3.0.

The amount of active ingredient (cetyl trimethyl ammonium chloride and lauryl alcohol) taken up varied from 6.5 to 7.6 g/m$^2$. Again, the weight ratio of cationic conditioning agent to fatty alcohol was 1:1.

Towels prepared using this combination of active ingredients were found to give a good conditioning effect but were less effective than the towels of Example 1.

Example 3

A liquid composition was prepared from the following ingredients, the pH being adjusted to 3.0:

| | Percentage by weight |
|---|---|
| "Arquad 16—50" | 5.50 |
| Sodium stearyl ($C_{18}$) sulphate (89% active) | 0.90 |
| Perfume | 0.60 |
| Formalin (38% active) | 0.26 |
| Hexacol orange dye (1% solution) | 0.75 |
| Water to | 100.0 |

Towels were prepared using this solution according to the procedure described in Example 1.

The total weight of the active ingredients taken up ranged from 4.52 to 5.20 g/m$^2$. The ratio of cationic conditioner to anionic conditioner was 3.43:1; thus the cationic conditioner content of the towels ranged from 3.50 to 4.02 g/m$^2$, and the anionic conditioner content ranged from 1.02 to 1.17 g/m$^2$.

These towels were found to have a conditioning effectiveness comparable to that of the towels of Example 2.

Example 4

A liquid composition was prepared from the following ingredients, the pH being adjusted to 3.0:

| | Percentage by weight |
|---|---|
| "Arquad 16—50" | 5.50 |
| Sodium cetyl ($C_{16}$) sulphate (30% active) | 2.46 |
| Perfume | 0.60 |
| Formalin (38% active) | 0.26 |
| Hexacol orange dye (1% solution) | 0.75 |
| Water to | 100.0 |

Towels were prepared using this solution according to the procedure described in Example 1.

The ratio of cationic conditioner to anionic conditioner was 3.73:1. The total conditioner deposited on each towel ranged from 4.48 to 5.31 g/m$^2$ consisting of 3.53 to 4.18 g/m$^2$ of cationic conditioner and 0.95 to 1.12 g/m$^2$ of anionic conditioner.

These towels showed outstanding conditioning performance, comparable to that of the towels of Example 1.

**Claims**

1. An article for conditioning hair, which comprises a flexible water-insoluble and non-water-dispersible substrate carrying a hair-conditioning agent, characterised in that the substrate is impregnated with an aqueous solution or dispersion comprising one or more hair-conditioning agents and colouring matter and/or perfume, the weight ratio of water associated with the substrate to the substrate being at least 1.5 to 1 and the total amount of hair-conditioning agent present being 2 to 15 g/m$^2$ of substrate, the impregnated substrate being sealed in a water-impermeable container.

2. An article as claimed in Claim 1, characterised in that it includes a water-soluble hair-conditioning agent.

3. An article as claimed in Claim 1 or Claim 2, characterised in that it includes a cationic hair-conditioning agent.

4. An article as claimed in Claim 3, characterised in that the cationic hair-conditioning agent is water-soluble and is present in dissolved form.

5. An article as claimed in Claim 3 or Claim 4, characterised in that the cationic hair-conditioning agent is a quaternary ammonium salt.

6. An article as claimed in Claim 5, characterised in that the quaternary ammonium salt is cetyl trimethyl ammonium chloride.

7. An article as claimed in any one of claims 3 to 6, characterised in that it includes as hair-conditioning agent a mixture of a cationic conditioning agent and a nonionic conditioning agent.

8. An article as claimed in any one of Claims 1 to 7, characterised in that it includes as hair-conditioning agent a fatty alcohol.

9. An article as claimed in Claim 8, characterised in that it includes as hair-conditioning agent lauryl, cetyl or stearyl alcohol.

10. An article as claimed in any one of Claims 1 to 6, characterised in that it includes as hair-conditioning agent a mixture of a cationic conditioning agent and an anionic conditioning agent.

11. An article as claimed in any preceding claim, characterised in that the total amount of hair-conditioning agent present is from 2 to 12 $g/m^2$ of substrate.

12. An article as claimed in Claim 11, characterised in that the total amount of hair-conditioning agent present is from 4 to 10 $g/m^2$ of substrate.

13. An article as claimed in Claim 12, characterised in that the total amount of hair-conditioning agent present is from 6 to 9 $g/m^2$ of substrate.

14. An article as claimed in any preceding claim, characterised in that the aqueous solution or dispersion with which the substrate is impregnated includes a minor proportion of a lower aliphatic alcohol.

15. An article as claimed in any preceding claim, characterised in that the water-impermeable container is a sachet of metal foil and/or plastics film.

16. A method for the manufacture of a hair-conditioning article as claimed in any one of Claims 1 to 15, characterised in that it comprises so impregnating a flexible water-insoluble and non-water-dispersible substrate with an aqueous solution or dispersion of one or more hair-conditioning agents, that in the impregnated article thus obtained the weight ratio of water associated with the substrate to the substrate is at least 1.5:1 and the total amount of hair-conditioning agent present is from 2 to 15 $g/m^2$ of substrate, and subsequently sealing the impregnated article in a water-impermeable container.

17. A method of conditioning hair, characterised in that it comprises rubbing dry, damp or wet hair with an impregnated substrate as claimed in any one of Claims 1 to 14, whereby an effective amount of hair-conditioning agent is transferred from the article to the hair.

**Patentansprüche**

1. Vorrichtung zum Konditionieren des Haares, die ein flexibles, wasserunlösliches und in Wasser nicht dispergierbares Substrat umfaßt, welches ein Haarkonditionierungsmittel trägt, dadurch gekennzeichnet, daß das Substrat mit einer wässrigen Lösung oder Dispersion imprägniert ist, die ein oder mehrere Haarkonditionierungsmittel und färbendes Material und/oder Parfum umfaßt, wobei das Gewichtsverhältnis von mit dem Substrat assoziertem Wasser zum Substrat wenigstens 1,5:1 und die Gesamtmenge an vorhandenem Haarkonditionierungsmittel 2 bis 15 $g/m^2$ Substrat ist, wobei das imprägnierte Substrat in einem wasser-undurchlässigem Behälter eingeschlossen ist.

2. Vorrichtung nach Anspruch 1, dadurch gekennzeichnet, daß sie ein wasserlösliches Haarkonditionierungsmittel enthält.

3. Vorrichtung nach Anspruch 1 oder Anspruch 2, dadurch gekennzeichnet, daß sie ein kationisches Haarkonditionierungsmittel enthält.

4. Vorrichtung nach Anspruch 3, dadurch gekennzeichnet, daß das kationische Haarkonditionierungsmittel wasserlöslich ist und in gelöster Form vorliegt.

5. Vorrichtung nach Anspruch 3 oder Anspruch 4, dadurch gekennzeichnet, daß das kationische Haarkonditionierungsmittel ein quaternäres Ammoniumsalz ist.

6. Vorrichtung nach Anspruch 5, dadurch gekennzeichnet, daß das quaternäre Ammoniumsalz Cetyltrimethylammoniumchlorid ist.

7. Vorrichtung nach irgendeinem der Ansprüche 3 bis 6, dadurch gekennzeichnet, daß sie als Haarkonditionierungsmittel ein Gemisch eines kationisches Konditionierungsmittels und eines nichtionischen Konditionierungsmittels enthält.

8. Vorrichtung nach irgendeinem der Ansprüche 1 bis 7, dadurch gekennzeichnet, daß sie als Haarkonditionierungsmittel einen Fettalkohol enthält.

9. Vorrichtung nach Anspruch 8, dadurch gekennzeichnet, daß sie als Haarkonditionierungsmittel Lauryl-, Cetyl- oder Stearylalkohol enthält.

10. Vorrichtung nach irgendeinem der Ansprüche 1 bis 6, dadurch gekennzeichnet, daß sie als

Haarkonditionierungsmittel ein Gemisch eines kationischen Konditionierungsmittels und eines anionischen Konditionierungsmittels enthält.

11. Vorrichtung nach irgendeinem vorhergehenden Anspruch, dadurch gekennzeichnet, daß die Gesamtmenge an vorhandenem Kaarkonditionierungsmittel 2 bis 12 g/m$^2$ Substrat ist.

12. Vorrichtung nach Anspruch 11, dadurch gekennzeichnet, daß die Gesamtmenge an vorhandenem Haarkonditionierungsmittel 4 bis 10 g/m$^2$ Substrat ist.

13. Vorrichtung nach Anspruch 12, dadurch gekennzeichnet, daß die Gesamtmenge an vorhandenem Haarkonditionierungsmittel 6 bis 9 g/m$^2$ Substrat ist.

14. Vorrichtung nach irgendeinem vorhergehenden Anspruch, dadurch gekennzeichnet, daß die wässrige Lösung oder Dispersion, mit der das Substrat imprägniert ist, eine geringere Menge eines niederen aliphatischen Alkohols enthält.

15. Vorrichtung nach irgendeinem vorhergehenden Anspruch, dadurch gekennzeichnet, daß der wasserundurchlässige Behälter ein Beutel aus Metallfolie und/oder Kunstfilm ist.

16. Verfahren zur Herstellung einer Haarkonditioniervorrichtung gemäß irgendeinem der Ansprüche 1 bis 15, dadurch gekennzeichnet, daß es das Imprägnieren eines flexiblen, wasserunlöslichen oder in Wasser nicht dispergierbaren Substrats mit einer wässrigen Lösung oder Dispersion eines oder mehrerer Haarkonditionierungsmittel umfaßt, daß in der so erhaltenen imprägnierten Vorrichtung das Gewichtsverhältnis von mit dem Substrat assoziiertem Wasser zum Substrat wenigstens 1,5:1 ist und die Gesamtmenge an vorhandenem Haarkonditionierungsmittel 2 bis 15 g/m$^2$ Substrat ist, und daß es das anschließende Einschließen der imprägnierten Vorrichtung in einem für Wasser undurchlässigen Behälter umfaßt.

17. Verfahren zum Konditionieren des Haares, dadurch gekennzeichnet, daß es das Reiben trockenen, feuchten oder nassen Haares mit einem imprägnierten Substrat gemäß irgendeinem der Ansprüche 1 bis 14 umfaßt, wodurch eine wirksame Menge Haarkonditionierungsmittel von der Vorrichtung auf das Haar übertragen wird.

**Revendications**

1. Article pour le conditionnement des cheveux qui comprend un substrat flexible, insoluble et non dispersible dans l'eau, portant un agent de conditionnement des cheveux, caractérisé en ce que le substrat est imprégné d'une solution ou dispersion aqueuse comprenant un ou plusieurs agents de conditionnement des cheveux, ainsi que des substances colorantes et/ou des parfums, le rapport en poids de l'eau associée avec le substrat, au substrat étant d'au moins 1,5 à 1, et la quantité totale présente d'agent de conditionnement des cheveux étant de 2 à 15 g/m$^2$ du substrat, le substrat imprégné étant scellé dans un conteneur imperméable à l'eau.

2. Article selon la revendication 1, caractérisé en ce qu'il renferme un agent soluble dans l'eau, de conditionnement des cheveux.

3. Article selon l'une des revendications 1 et 2, caractérisé en ce qu'il renferme un agent cationique de conditionnement des cheveux.

4. Article selon la revendication 3, caractérisé en ce que l'agent cationique de conditionnement des cheveux est soluble dans l'eau, et en ce qu'il est présent sous forme dissoute.

5. Article selon l'une des revendications 3 et 4, caractérisé en ce que l'agent cationique de conditionnement des cheveux est un sel d'ammonium quaternaire.

6. Article selon la revendication 5, caractérisé en ce que le sel d'ammonium quaternaire est le chlorure de cétyl-triméthyl-ammonium.

7. Article selon l'une des revendications 3 à 6, caractérisé en ce qu'il renferme, comme agent de conditionnement des cheveux, un mélange d'un agent cationique de conditionnement et d'un agent non ionique de conditionnement.

8. Article selon l'une des revendication 1 à 7, caractérisé en ce qu'il comprend comme agent de conditionnement des cheveux, un alcool gras.

9. Article selon la revendication 8, caractérisé en qu'il renferme comme agent de conditionnement des cheveux, l'alcool laurique, cétylique ou stéarique.

10. Article selon l'une des revendications 1 à 6, caractérisé en ce qu'il comprend, comme agent de conditionnement des cheveux, un mélange d'un agent cationique de conditionnement et d'un agent anionique de conditionnement.

11. Article selon l'une des revendications 1 à 10, caractérisé en ce que la quantité totale présente d'agent de conditionnement des cheveux, est comprise entre 2 et 12 g/m$^2$ du substrat.

12. Article selon la revendication 11, caractérisé en ce que la quantité totale présente d'agent de conditionnement des cheveux, est comprise entre 4 et 10 g/m$^2$ de substrat.

13. Article selon la revendication 12, caractérisé en ce que la quantité totale présente d'agent de conditionnement des cheveux, est comprise entre 6 et 9 g/m$^2$ de substrat.

14. Article selon l'une des revendications 1 à 13, caractérisé en ce que la solution ou dispersion aquese dont le substrat est imprégné, renferme un faible proportion d'un alcool aliphatique inférieur.

15. Article selon l'une des revendications 1 à 14, caractérisé en ce que le conteneur imperméable à l'eau est un sachet composé d'une feuille métallique et/ou d'une pellicule de matière plastique.

16. Méthode de fabrication d'un article pour le conditionnement des cheveux selon l'une des revendications 1 à 15, caractérisée en ce qu'on imprègne suffisamment un substrat flexible, insoluble et non dispersible dans l'eau, d'une solution ou dispersion aqueuse d'un ou plusieurs agents de conditionnement des cheveux, pour que dans l'article imprégné ainsi obtenu le rapport en poids de l'eau associée avec le substrat, au substrat, soit d'au moins 1,5:1 et pour que la quantité totale présente d'agent de conditionnement des cheveux soit de 2 à 15 g/m$^2$ de substrat, l'article imprégné étant ultérieurement scellé dans un conteneur imperméable à l'eau.

17. Méthode pour le conditionnement des cheveux, caractérisée en ce qu'elle consiste à frotter des cheveux secs, humides ou mouillés avec un substrat imprégné selon l'une des revendications 1 à 14, une quantité efficace de l'agent de conditionnement des cheveux étant ainsi transférée de l'article sur les cheveux.